# Europäisches Patentamt
# European Patent Office
# Office européen des brevets

(19)

(11) Veröffentlichungsnummer: **0 052 247**
**B1**

(12)

# EUROPÄISCHE PATENTSCHRIFT

(45) Veröffentlichungstag der Patentschrift:
**27.02.85**

(51) Int. Cl.⁴: **A 61 N 1/36**

(21) Anmeldenummer: **81108569.5**

(22) Anmeldetag: **20.10.81**

(54) Vorrichtung zur elektrischen Stimulation von Knochen im Frakturbereich.

(30) Priorität: **13.11.80 DE 3042751**

(43) Veröffentlichungstag der Anmeldung:
**26.05.82 Patentblatt 82/21**

(45) Bekanntmachung des Hinweises auf die Patenterteilung:
**27.02.85 Patentblatt 85/9**

(84) Benannte Vertragsstaaten:
**CH DE FR GB IT LI SE**

(56) Entgegenhaltungen:
**DE - A - 2 552 523**
**DE - A - 2 558 525**
**DE - B - 2 147 704**
**GB - A - 2 053 687**
**US - A - 3 672 352**
**US - A - 3 924 641**
**US - A - 3 968 802**

(73) Patentinhaber: **Jorgensen, Torben Ejsing, Dr., 8, Egevangen, DK-8900 Randers (DK)**

(72) Erfinder: **Jorgensen, Torben Ejsing, Dr., 8, Egevangen, DK-8900 Randers (DK)**

(74) Vertreter: **Hauck, Hans, Dipl.-Ing. et al, Patentanwälte Dipl.-Ing. H. Hauck Dipl.-Phys. W. Schmitz Dipl.-Ing. E. Graalfs Dipl.-Ing. W. Wehnert - Dr.-Ing. W. Döring, Neuer Wall 41 D-2000 Hamburg 36 (DE)**

## Beschreibung

Es ist bekannt, dass bei der Belastung von Knochen und bei der Osteogenese im Knochen elektrische Potentiale erzeugt werden. Es ist eine Reihe von Versuchen angestellt worden, im zu behandelnden Gebiet des Knochens von extern ein Potential zu erzeugen, um die Osteogenese zu stimulieren. Die elektrische Stimulation von Knochen wird angewandt sowohl bei frischen Brüchen als auch bei Pseudoarthrose oder anderen Defekten, welche dem Ausheilen eines Bruches im Wege sind. Es ist bekannt, ein eine positive Elektrode bildendes Gehäuse, das eine Gleichstromquelle aufnimmt, im weichen Gewebe oder medullär nahe der Stelle der Verletzung zu implantieren und eine negative Elektrode an der Stelle, an der die Knochenbildung stattfinden soll, anzusetzen (DE-A Nr. 2552523). Zwischen den Elektroden liegen 1,3 bis 9 V Gleichspannung an. Die Anwendung konstanter Gleichspannung hat sich jedoch als unvorteilhaft erwiesen, insbesondere im Hinblick auf die Beeinträchtigung des Gewebes durch Korrosion. Auch das gewünschte Wachstum wird durch stetigen Gleichstrom nicht in ausreichendem Masse stimuliert. Das Implantieren der Elektrode mit der Spannungsquelle erfordert zwei operative Eingriffe, die eine Infektionsgefahr mit sich bringen. Eine Überwachung der implantierten Stimulationsvorrichtung über eine längere Behandlungsdauer ist kaum möglich.

Der Erfindung liegt die Aufgabe zugrunde, eine Vorrichtung zur elektrischen Stimulation von Knochen zu schaffen, die eine besonders wirksame Heilung von Knochenbrüchen ermöglicht, keine Beeinträchtigung von Knochen und Geweben verursacht und dem Patienten eine weitgehende Bewegungsmöglichkeit lässt, ohne dass eine strenge Überwachung durch ärztliches Personal oder den Patienten erforderlich ist.

Diese Aufgabe wird durch die Merkmale des Anspruchs 1 gelöst.

Durch die Metallstifte der externen Fixationsvorrichtung wird die Spannung unmittelbar über dem Bruch am Knochen angelegt. Das Stimulationssignal kann daher unmittelbar auf die Bruchfläche einwirken.

Die Anordnung der elektrischen bzw. elektronischen Komponenten in einer länglichen Hülse ermöglicht die einfache Anbringung dieser Hülse an der externen Fixationsvorrichtung, wobei durch flexible Kabel eine elektrische Verbindung zu den Metallstiften geschaffen ist. Der Patient ist daher während der sich über mehrere Monate hinstreckenden Behandlungsdauer nicht an das Bett gefesselt, sondern kann sich mehr oder weniger frei bewegen. Da die Umpoleinrichtung über den Impulszähler automatisch gesteuert ist, bedarf die Behandlung keiner ständigen Überwachung durch ärztliches Personal oder den Patienten. Es ist lediglich erforderlich, von Zeit zu Zeit zu überprüfen, ob der Stimulator noch im Betrieb ist und in gewünschter Weise eine Vorzeichenumkehr der Impulse herbeiführt, was z.B. alle 2 h geschieht.

Ein Ausführungsbeispiel nach der Erfindung wird nachfolgend anhand von Zeichnungen näher erläutert.

Fig. 1 zeigt perspektivisch eine Fixationsvorrichtung nach Hoffmann mit einer Vorrichtung nach der Erfindung.

Fig. 2 zeigt eine abgewandelte gekoppelte Schaltung nach der Erfindung.

Fig. 3 zeigt in Blockdarstellung eine Schaltungsanordnung für eine Vorrichtung nach der Erfindung.

Fig. 4 zeigt ein Impulsdiagramm, das mit Hilfe eines Stimulators nach Fig. 3 erzeugt wird.

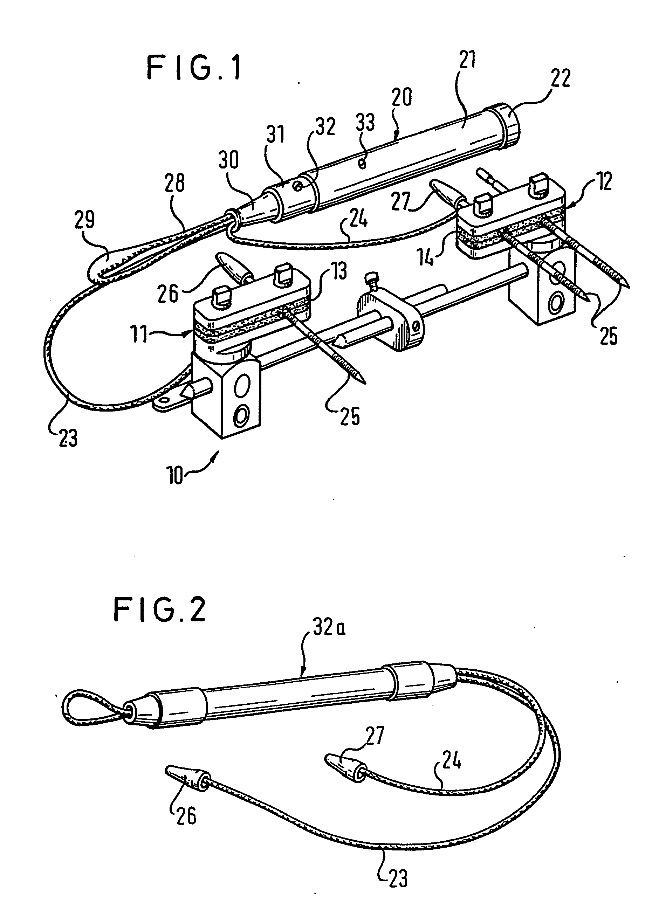

Die in Fig.1 dargestellte Fixationsvorrichtung 10 ist allgemein bekannt und soll daher im einzelnen nicht beschrieben werden. Sie besitzt zwei relativ zueinander bewegbare Einspannvorrichtungen 11, 12, welche im wesentlichen aus Spannbacken bestehen, deren eigentliche Spannglieder aus isolierendem Material bestehen, wie bei 13 bzw. 14 dargestellt. In den Einspannvorrichtungen 11, 12 werden Stifte 25 eingespannt, nachdem sie in ein entsprechendes Knochenfragment eingebohrt bzw. eingeschraubt worden sind. Die Spannvorrichtungen können einen oder mehrere Stifte 25 an verschiedenen Stellen einspannen, wie das etwa bei der Einspannvorrichtung 12 dargestellt ist. Durch die isolierte Einspannung der Stifte 25 sind diese elektrisch gegeneinander isoliert.

Ein Stimulator, wie er in Verbindung mit der Fixationsvorrichtung 10 verwendet werden kann, ist allgemein mit 20 bezeichnet. Sein Gehäuse ist eine längliche, im Querschnitt kreisförmige Aluminiumhülse 21, welche an einem Ende mittels einer Kappe 22 verschlossen ist. An der Stirnseite sind aus der Hülse 21 zwei flexible Kabel 23, 24 herausgeführt, die an den Enden mit Steckbuchsen 26, 27 versehen sind, welche auf die hinteren Enden der Stifte 25 gesteckt werden können. Ferner ist am gleichen Ende der Hülse 21 ein weiteres, etwas kürzeres flexibles Kabel 28 herausgeführt, in dem in einer Verdickung 29 Leuchtdioden angeordnet sind. Eine isolierende Schutzkappe 30 ist mit Hilfe einer Klemmbuchse 31 und einer Schraube 32 vor das entsprechende Ende der Hülse 21 gesetzt.

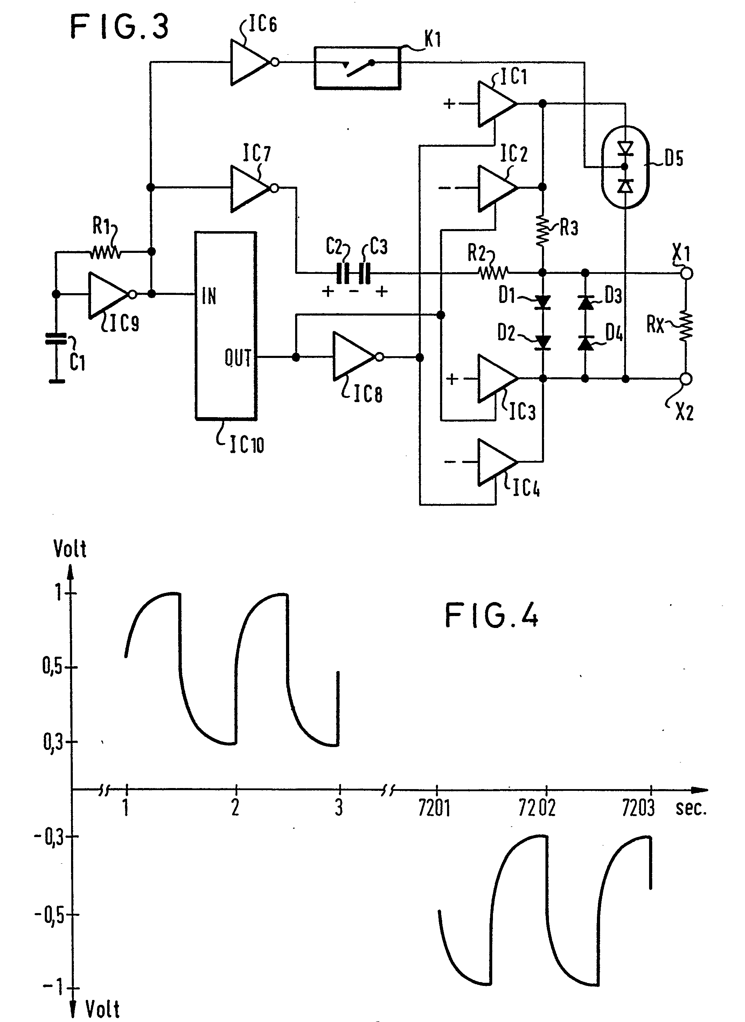

Eine Patrone 32a ist in Fig. 2 dargestellt. Sie weist ein metallisches Gehäuse auf, beispielsweise aus Aluminium, in dem eine Lithiumbatterie und eine miniaturisierte elektronische Schaltungsanordnung angeordnet ist. Die elektronische Schaltungsanordnung ist im Gehäuse der Lithiumbatterie untergebracht. Die im Gehäuse 20 (Fig. 1) bzw. in der Patrone 32 angeordnete elektronische Schaltung ist – als Beispiel – in Fig. 3 wiedergegeben.

Die Ausgangsklemmen der Schaltungsanordnung in Fig. 3 sind mit X1 und X2 bezeichnet. Sie sind mit den in den Fig. 1 und 2 dargestellten Leiterkabeln 23, 24 verbunden. Das an ihnen anstehende Potential ist mithin das zwischen den Knochenfragmenten anliegende Potential. Über einen Prüfverstärker IC7, Kondensatoren C2, C3 und einem Widerstand R2 liegt die Klemme X1 an einen Impulsgenerator, der durch das Bauteil IC9 repräsentiert wird. Der Rückkopplungseingang über R1 liegt über Kondensator C1 an Masse.

Wie aus Fig. 3 ferner hervorgeht, liegen die Ausgangsklemmen X1, X2 jeweils am Ausgang elektronischer Schalter IC1, IC2 bzw. IC3 und IC4. Der Eingang der elektronischen Schalter ist jeweils mit einem Pol einer Lithiumbatterie verbunden, wie dies lediglich durch die Vorzeichen + und − angedeutet ist. Die Steuereingänge der elektronischen Schalter IC2 und IC3 liegen unmittelbar am Ausgang eines Impulszählers IC10, während die Steuereingänge der elektronischen Schalter IC1 und IC4 über einen Inverter IC8 ebenfalls am Ausgang des Impulszählers IC10 liegen. Der Eingang des Impulszählers IC10 ist mit dem Ausgang des Impulsgenerators IC9 verbunden. Eine erste Reihenschaltung aus zwei Dioden D1 und D2 liegt parallel zu den Klemmen X1, X2. Eine zweite Reihenschaltung von Dioden D3 und D4 liegt ebenfalls parallel zu den Klemmen X1 und X2, hat jedoch die entgegengesetzte Durchlassrichtung. Der Ausgang des Impulsgenerators IC9 liegt ferner über einen Pufferverstärker IC6 an einer Klemme eines Reed-Kontaktes K1, dessen andere Klemme an einem Punkt zwischen zwei Leuchtdioden geschaltet ist, die zusammen mit D5 bezeichnet sind. Die Anode der unteren Leuchtdiode ist mit der Klemme X2 verbunden, während die Anode der oberen Leuchtdiode mit den Ausgängen der elektronischen Schaltung IC1 und IC2 verbunden ist.

Die beschriebene Schaltungsanordnung arbeitet wie folgt. Unabhängig davon, ob der Ausgang des Impulszählers hoch oder niedrig ist, legt ein Paar der elektronischen Schalter IC1 bis IC4 eine Gleichspannung an die Klemmen X1, X2, und zwar über den Widerstand R3. Diese Gleichspannung wird nun nach Massgabe der Impulse des Impulsgenerators IC9 moduliert, wobei über die Kondensatoren C2 und C3 und den Widerstand R2 eine Impulsformung vorgenommen wird, derart, wie sie in Fig. 4 dargestellt ist. Es entstehen asymmetrische Impulse, wie sie in Fig. 4 dargestellt sind. Es hat sich gezeigt, dass derartige asymmetrische Impulse für die Therapie besonders günstig sind. Die Frequenz beträgt 1 Hz und die Amplitude annähernd 0,7 V, während die Spitzenspannung 1 V beträgt. Die in Reihe geschalteten Siliziumdioden D1 bis D4 stellen sicher, dass die Spannung nicht grösser als 1 bis 1,6 V wird, eine Spannung, bei der die Durchlassspannung der in Reihe liegenden Dioden erreicht ist. Im übrigen stellen die Dioden D1 bis D4 gleichzeitig eine Spannungsbegrenzung hinsichtlich solcher Potentiale dar, welche von aussen an die Klemmen X1, X2 gelangen, und verhindern mithin die Beschädigung von Schaltungsteilen. Der Impulszähler IC10 ist auf eine bestimmte Impulszahl eingestellt, beispielsweise auf $2^{14}$ Impulse, was bei der Frequenz von 1 Hz einer Zeit von 2 h entspricht. Daher erfolgt alle 2 h über die elektronischen Schalter IC1 bis IC4 eine Vorzeichenumkehr der pulsierenden Gleichspannung an den Klemmen X1 und X2.

Wird der Reed-Kontakt K1 mit Hilfe eines externen in die Nähe gebrachten Magneten etwa in die Nähe der Schraube 33 geschlossen, leuchtet — je nach Vorzeichen der pulsierenden Gleichspannung — eine der beiden Leuchtdioden auf und zeigt, ob der Stimulator in Betrieb ist und welches Vorzeichen die Stimulatorspannung hat.

Die erfindungsgemässe Vorrichtung wurde in Verbindung mit einer Fixationsvorrichtung nach Hoffmann beschrieben. Es versteht sich, dass auch andere Fixationsmittel zur Anwendung gelangen können, beispielsweise Steinmann-Pins oder ähnliches.

Die Stimulierung mit der Vorrichtung nach Hoffmann hat den grossen Vorteil, dass das Potential über die Bruchstelle angelegt wird. Dies hat sich als besonders günstig für die Heilung erwiesen.

## Patentansprüche

1. Vorrichtung zur elektrischen Stimulation von Knochen im Frakturbereich, mit zwei durch in die Bruchfragmente einführbare Metallstifte (25) gebildeten Elektroden, die isoliert in einer externen Fixationsvorrichtung (10) eingespannt sind; mit einer Spannungsquelle, die eine Impulsfolge mit einer Impulsfolgefrequenz von etwa 1 Hz und etwa 1 V Spannung sowie eine den Impulsen unterlegte, konstante Gleichspannung erzeugt; mit einem mit einer Impulsquelle (IC9) verbundenen Impulszähler (IC10), der ein Umpolsignal für eine Umpoleinrichtung (IC1 bis IC4) abgibt, wenn die Zahl der Impulse einen vorgegebenen Wert erreicht, und mit einem länglichen Gehäuse (20), in dem Impulszähler (IC10), Spannungsquelle (IC9) und Umpoleinrichtung (IC1 bis IC4) untergebracht sind, und das über flexible Zuleitungen (23, 24) mit den Metallstiften (25) verbunden ist.

2. Vorrichtung nach Anspruch 1, dadurch gekennzeichnet, dass die Umpoleinrichtung (IC1 bis IC4) ein Umpolsignal abgibt, wenn die Zahl der Impulse einer Zeitspanne von etwa 2 h entspricht.

3. Vorrichtung nach einem der Ansprüche 1 oder 2, dadurch gekennzeichnet, dass eine Anzeigevorrichtung (D5) mittels eines Kontrollschalters (K1) wahlweise mit den Elektroden (25) verbunden wird.

4. Vorrichtung nach einem der Ansprüche 1 bis 3, dadurch gekennzeichnet, dass zwischen die Elektroden (X1, X2) eine Spannungsbegrenzungsvorrichtung (D1 bis D4) geschaltet ist.

5. Vorrichtung nach Anspruch 4, dadurch gekennzeichnet, dass eine oder zwei in Reihe geschaltete Dioden (D1 bis D4) antiparallel zwischen die Elektroden (X1, X2) geschaltet sind.

6. Vorrichtung nach einem der Ansprüche 1 bis 5, dadurch gekennzeichnet, dass zwei Leuchtdioden mit jeweils umgekehrter Durchlassrichtung geschaltet sind, die Reihenschaltung (D5) der Leuchtdioden parallel zum Ausgang der Umpoleinrichtung (IC1 bis IC4) geschaltet ist und der Kontrollschalter (K1) zwischen der Impulsquelle (IC9) und der Verbindung zwischen den beiden Leuchtdioden (D5) geschaltet ist.

7. Vorrichtung nach Anspruch 6, dadurch gekennzeichnet, dass der Kontrollschalter (K1) ein Reed-Kontakt ist.

8. Vorrichtung nach einem der Ansprüche 1 bis 7, dadurch gekennzeichnet, dass die Gleichspannungsquelle eine Lithiumbatterie ist.

9. Vorrichtung nach einem der Ansprüche 1 bis 8, dadurch gekennzeichnet, dass das längliche Gehäuse (21) eine Aluminiumhülse ist.

10. Vorrichtung nach einem der Ansprüche 1 bis 9, dadurch gekennzeichnet, dass die Anzeigevorrichtung (D5) mit einer weiteren, aus dem Ende des Gehäuses (21) herausgeführten flexiblen Zuleitung (28) verbunden ist.

11. Vorrichtung nach einem der Ansprüche 7 oder 10, dadurch gekennzeichnet, dass das Gehäuse (21) aus unmagnetischem Material besteht, dass der Reed-Kontakt ebenfalls im Gehäuse (21) angeordnet ist, und dass ein Permanentmagnet beweglich mit dem Gehäuse (21) verbunden ist.

12. Vorrichtung nach Anspruch 11, dadurch gekennzeichnet, dass der Permanentmagnet mit der Zuleitung für die Anzeigevorrichtung verbunden ist.

13. Vorrichtung nach Anspruch 1, dadurch gekennzeichnet, dass die Amplitude annähernd 0,7 V beträgt.

## Revendications

1. Dispositif destiné à la stimulation électrique d'os dans la zone de fracture, comportant deux électrodes constituées par des broches métalliques (25) insérables dans les fragments de la cassure et qui sont encastrées de manière isolée dans un dispositif extérieur de fixation (10), une source de tension qui produit une suite d'impulsions d'une fréquence d'environ 1 Hz et d'une tension d'environ 1 V, ainsi qu'une tension continue constante sous-jacente aux impulsions, un compteur d'impulsions (IC10) relié à une source d'impulsions (IC9) et qui délivre un signal inverseur destiné à un dispositif d'inversion (IC1 à IC4) lorsque le nombre des impulsions a atteint une valeur prédéterminée, et un boîtier allongé (20) où sont disposés le compteur d'impulsions (IC10), la source de tension (IC9) et le dispositif d'inversion (IC1 à IC4) et qui est relié par des conducteurs flexibles (23, 24) aux broches métalliques (25).

2. Dispositif selon la revendication 1, caractérisé en ce que le dispositif d'inversion (IC1 à IC4) délivre un signal inverseur lorsque le nombre des impulsions correspond à un intervalle de temps d'environ 2 h.

3. Dispositif selon l'une des revendications 1 ou 2, caractérisé en ce qu'un dispositif d'affichage (D5) est relié au moyen d'un commutateur de contrôle (K1) sélectivement aux électrodes (25).

4. Dispositif selon l'une des revendications 1 à 3, caractérisé en ce qu'entre les électrodes (X1, X2) est monté un dispositif de limitation de tension (D1 à D4).

5. Dispositif selon la revendication 4, caractérisé en ce qu'une ou deux diodes montées en série (D1 à D4) sont montées en opposition entre les électrodes (X1 et X2).

6. Dispositif selon l'une des revendications 1 à 5, caractérisé en ce que deux diodes luminescentes sont montées avec un sens de passage respectivement inversé, en ce que le montage série (D5) des diodes luminescentes se trouve en parallèle sur la sortie du dispositif d'inversion (IC1 à IC4) et en ce que le commutateur de contrôle (K1) est monté entre la source d'impulsions (IC9) et la liaison entre les deux diodes luminescentes (D5).

7. Dispositif selon la revendication 6, caractérisé en ce que le commutateur de contrôle (K1) est un contact à lames.

8. Dispositif selon l'une des revendications 1 à 7, caractérisé en ce que la source de tension continue est une pile au lithium.

9. Dispositif selon l'une des revendications 1 à 8, caractérisé en ce que le boîtier allongé (21) est une cartouche en aluminium.

10. Dispositif selon l'une des revendications 1 à 9, caractérisé en ce que le dispositif d'affichage (D5) est relié à un autre conducteur (28) flexible sortant de l'extrémité du boîtier (21).

11. Dispositif selon l'une des revendications 7 ou 10, caractérisé en ce que le boîtier (21) est fait d'une matière non magnétique, en ce que le contact à lames est disposé également dans le boîtier (21) et en ce qu'un aimant permanent est relié de façon mobile au boîtier (21).

12. Dispositif selon la revendication 11, caractérisé en ce que l'aimant permanent est relié au conducteur destiné au dispositif d'affichage.

13. Dispositif selon la revendication 1, caractérisé en ce que l'amplitude de la tension est d'environ 0,7 V.

## Claims

1. An apparatus for the electric stimulation of bones in the zone of a fracture, comprising two electrodes formed by metal pins (25) insertable into the fragments of the fracture, said electrodes being clamped in an external fixation device (10) in an insulated manner; a voltage source which generates a sequence of pulses with a pulse sequence frequency of about 1 Hz and a voltage of about 1 V as well as a constant d.c. voltage subjected to the pulses; a pulse counter (IC10) connected to a pulse source (IC9) providing a polarity inversion signal for a polarity inversion means (IC1 to IC4) when the number of pulses reaches a predetermined value; and an elongated housing (20) with the pulse counter (IC10), voltage source (IC9) and polarity inversion means (IC1 to IC4) accommodated therein, said housing being connected to the metal pins (25) via flexible supply lines (23, 24).

2. An apparatus according to Claim 1, characterised in that the polarity inversion means (IC1 to IC4) provides a polarity inversion signal when the number of pulses corresponds to a period of time of about 2 h.

3. An apparatus according to Claim 1 or 2, characterised in that an indicating means (D5) is selectively connected to the electrodes (25) by means of a control switch (K1).

4. An apparatus according to any of Claims 1 to 3, characterised in that a voltage limiting device (D1 to D4) is interposed between the electrodes (X1, X2).

5. An apparatus according to Claim 4, characterised in that one or two series-connected diodes (D1 to D4) are interposed between the electrodes (X1, X2) in anti-parallel arrangement.

6. An apparatus according to any of Claims 1 to 5, characterised in that two light-emissive diodes are switched respectively to have an inverted direction of passage, the series-connection (D5) of the light-emissive diodes being connected in parallel with the output of the polarity inversion means (IC1 to IC4) and the control switch (K1) being connected between the pulse source (IC9) and the connection between the two light-emissive electrodes (D5).

7. An apparatus according to Claim 6, characterised in that the control switch (K1) is a Reed contact.

8. An apparatus according to any of Claims 2 to 7, characterised in that the d.c. source is a lithium battery.

9. An apparatus according to any of Claims 1 to 8, characterised in that the elongated housing (21) is an aluminum sleeve.

10. An apparatus according to any of Claims 1 to 9, characterised in that the indicating means (D5) is connected to a further flexible supply line (28) exiting from the end of the housing (21).

11. An apparatus according to Claim 7 or 10, characterised in that the housing (21) consists of non-magnetic material, the Reed contact being likewise arranged in the housing (21) and a permanent magnet being movably connected to the housing (21).

12. An apparatus according to Claim 11, characterised in that the permanent magnet is connected to the supply line for the indicating means.

13. An apparatus according to Claim 1, characterised in that the amplitude is approximately 0.7 V.

FIG.1

FIG.2

FIG.3

FIG.4